# NEW EUROPEAN PATENT SPECIFICATION

(11) **EP 1 781 814 B3**
(45) Date of publication of this specification: **31.08.2011**
(45) Mention of the grant of the patent: 06.01.2010
(21) Application number: 05763127.7
(22) Date of filing: 27.07.2005
(51) Int. Cl.: C12Q 1/68

(54) **METHODS AND KIT FOR THE PROGNOSIS OF BREAST CANCER**
VERFAHREN UND KIT ZUR PROGNOSE VON BRUSTKREBS
PROCEDES ET TROUSSE POUR LE PRONOSTIC DU CANCER DU SEIN

(30) Priority: 10.08.2004 GB 0417740; 07.12.2004 GB 0426777
(43) Date of publication of application: 09.05.2007
(60) Divisional application: 08019419.4
(73) Proprietor: Cardiff Biologicals Limited, Cardiff CF24 4AY (GB)
(72) Inventor: JIANG, Wen Guo, University Department of Surgery, Cardiff CF14 4XN (GB)
(74) Representative: Fyles, Julie Marie
(86) International application number: PCT/GB2005/002971
(87) International publication number: WO 2006/016110

(56) References cited:
- EP-A- 1 435 243
- WO-A-99/38500
- WO-A-02/059377
- WO-A-03/050307
- WO-A-03/054165
- WO-A-03/083141
- US-A- 5 872 217
- US-A- 5 935 798
- US-A1- 2003 064 384
- US-A1- 2003 175 855
- US-A1- 2004 086 504
- SCHWIRZKE MARINA ET AL: "Identification of metastasis-associated genes by transcriptional profiling of a pair of metastatic versus non-metastatic human mammary carcinoma cell lines" ANTICANCER RESEARCH, vol. 21, no. 3B, May 2001 (2001-05), pages 1771-1776, XP002967438 ISSN: 0250-7005
- TSUTSUMI SOICHI ET AL: "Overexpression of the autocrine motility factor/phosphoglucose isomerase induces transformation and survival of NIH-3T3 fibroblasts." CANCER RESEARCH, vol. 63, no. 1, 1 January 2003 (2003-01-01), pages 242-249, XP002349071 ISSN: 0008-5472
- PARR CHRISTIAN ET AL: "The hepatocyte growth factor regulatory factors in human breast cancer." CLINICAL CANCER RESEARCH : AN OFFICIAL JOURNAL OF THE AMERICAN ASSOCIATION FOR CANCER RESEARCH. 1 JAN 2004, vol. 10, no. 1 Pt 1, 1 January 2004 (2004-01-01), pages 202-211, XP002371735 ISSN: 1078-0432
- FIRON MICHAL ET AL: "Dominant negative Met reduces tumorigenicity-metastasis and increases tubule formation in mammary cells" ONCOGENE, vol. 19, no. 20, 11 May 2000 (2000-05-11), pages 2386-2397, XP002371736 ISSN: 0950-9232
- DRESSMAN M A ET AL: "Gene expression profiling detects gene amplification and differentiates tumor types in breast cancer" CANCER RESEARCH, AMERICAN ASSOCIATION FOR CANCER RESEARCH, BALTIMORE, MD, US, vol. 63, no. 9, 1 May 2003 (2003-05-01), pages 2194-2199, XP002317347 ISSN: 0008-5472
- ALPY FABIEN ET AL: "Metastatic lymph node 64 (MLN64), a gene overexpressed in breast cancers, is regulated by Sp/KLF transcription factors." ONCOGENE, vol. 22, no. 24, 12 June 2003 (2003-06-12), pages 3770-3780, XP002371737 ISSN: 0950-9232
- ROSS J S ET AL: "Co-expression of matrilysin (MMP7) and stromelysin (MMPs3, 10, and 11) proteins and prognostic significance in mammary carcinomas" LABORATORY INVESTIGATION, vol. 82, no. 1, January 2002 (2002-01), page 50A, XP008061400 & 2002 ANNUAL MEETING OF THE UNITED STATES AND CANADIAN ACADEMY OF PATHOLOGY; CHICAGO, IL, USA; FEBRUARY 23-MARCH 01, 2002 ISSN: 0023-6837
- FABRE STEPHANIE ET AL: "Prominent role of the Ig-like V domain in trans-interactions of nectins. Nectin3 and nectin4 bind to the predicted C-C'-C''-D beta-strands of the nectin1 V domain" JOURNAL OF BIOLOGICAL CHEMISTRY, vol. 277, no. 30, 28 July 2002 (2002-07-28), pages 27006-27013, XP002371738 ISSN: 0021-9258
- SPICER JAMES ET AL: "Regulation of the Wnt signalling component PAR1A by the Peutz-Jeghers syndrome kinase LKB1." ONCOGENE. 24 JUL 2003, vol. 22, no. 30, 24 July 2003 (2003-07-24), pages 4752-4756, XP002371739 ISSN: 0950-9232
- JIANG WEN G ET AL: "Psoriasin is aberrantly expressed in human breast cancer and is related to clinical outcomes" INTERNATIONAL JOURNAL OF ONCOLOGY, vol. 25, no. 1, July 2004 (2004-07), pages 81-85, XP008061313 ISSN: 1019-6439
- BERNAL JUAN A ET AL: "Human securin interacts with p53 and modulates p53-mediated transcriptional activity and apoptosis" NATURE GENETICS, vol. 32, no. 2, October 2002 (2002-10), pages 306-311, XP002371740 ISSN: 1061-4036
- KLEER CELINA G ET AL: "Characterization of RhoC expression in benign and malignant breast disease: A potential new marker for small breast carcinomas with metastatic ability" AMERICAN JOURNAL OF PATHOLOGY, vol. 160, no. 2, February 2002 (2002-02), pages 579-584, XP002371741 ISSN: 0002-9440
- BOURDON J -C ET AL: "Scotin, a novel p53-inducible proapoptotic protein located in the ER and the nuclear membrane" JOURNAL OF CELL BIOLOGY, vol. 158, no. 2, 22 July 2002 (2002-07-22), pages 235-246, XP002371742 ISSN: 0021-9525
- LEE BYEONG-CHEL ET AL: "Involvement of the chemokine receptor CXCR4 and its ligand stromal cell-derived factor 1alpha in breast cancer cell migration through human brain microvascular endothelial cells" MOLECULAR CANCER RESEARCH, vol. 2, no. 6, June 2004 (2004-06), pages 327-338, XP002371743 ISSN: 1541-7786
- SWISSHELM K ET AL: "SEMP1, a senescence-associated cDNA isolated from human mammary epithelial cells, is a member of an epithelial membrane protein superfamily" GENE, ELSEVIER, AMSTERDAM, NL, vol. 226, no. 2, 21 January 1999 (1999-01-21), pages 285-295, XP004155143 ISSN: 0378-1119
- SAMPATH JANARDHAN ET AL: "SPF45, a novel spliceosome-associated protein is overexpressed in tumors" PROCEEDINGS OF THE AMERICAN ASSOCIATION FOR CANCER RESEARCH ANNUAL MEETING, vol. 43, March 2002 (2002-03), page 140, XP008061428 & 93RD ANNUAL MEETING OF THE AMERICAN ASSOCIATION FOR CANCER RESEARCH; SAN FRANCISCO, CALIFORNIA, USA; APRIL 06-10, 2002 ISSN: 0197-016X
- SCHULZ SOLVEIG ET AL: "True positive somatostatin receptor scintigraphy in primary breast cancer correlates with expression of sst2A and sst5." BREAST CANCER RESEARCH AND TREATMENT. APR 2002, vol. 72, no. 3, April 2002 (2002-04), pages 221-226, XP002371744 ISSN: 0167-6806
- LAUFFART BRENDA ET AL: "Molecular cloning, genomic structure and interactions of the putative breast tumor suppressor TACC2." GENOMICS, vol. 81, no. 2, February 2003 (2003-02), pages 192-201, XP002371748 ISSN: 0888-7543
- DAVIES GAYNOR ET AL: "Levels of expression of endothelial markers specific to tumour-associated endothelial cells and their correlation with prognosis in patients with breast cancer." CLINICAL & EXPERIMENTAL METASTASIS. 2004, vol. 21, no. 1, 2004, pages 31-37, XP002371749 ISSN: 0262-0898
- WITTLIFF JAMES L: "Molecular signatures of endocrine responsive breast cancer using gene expression profiling." JOURNAL OF CLINICAL LIGAND ASSAY, vol. 26, no. 1, April 2003 (2003-04), pages 45-49, XP008061401 ISSN: 1081-1672
- ADEYINKA ADEWALE ET AL: "Analysis of gene expression in ductal carcinoma in situ of the breast." CLINICAL CANCER RESEARCH : AN OFFICIAL JOURNAL OF THE AMERICAN ASSOCIATION FOR CANCER RESEARCH. DEC 2002, vol. 8, no. 12, December 2002 (2002-12), pages 3788-3795, XP002383037 ISSN: 1078-0432
- FUNASAKA TATSUYOSHI ET AL: "Tumor autocrine motility factor induces hyperpermeability of endothelial and mesothelial cells leading to accumulation of ascites fluid" BIOCHEMICAL AND BIOPHYSICAL RESEARCH COMMUNICATIONS, vol. 293, no. 1, 26 April 2002 (2002-04-26), pages 192-200, XP002383038 ISSN: 0006-291X
- GOODISON STEVE ET AL: "The interrelationship between DRIM gene expression and cytogenetic and phenotypic characteristics in human breast tumor cell lines." BMC GENOMICS, vol. 4, no. 39 Cited October 17, 2003, 22 September 2003 (2003-09-22), XP002383039 ISSN: 1471-2164
- OKTAY MAJA H ET AL: "Focal adhesion kinase as a marker of malignant phenotype in breast and cervical carcinomas." HUMAN PATHOLOGY. MAR 2003, vol. 34, no. 3, March 2003 (2003-03), pages 240-245, XP002383040 ISSN: 0046-8177
- ARIGA NAOHIRO ET AL: "Stromal expression of fibroblast activation protein/seprase, a cell membrane serine proteinase and gelatinase, is associated with longer survival in patients with invasive ductal carcinoma of breast" INTERNATIONAL JOURNAL OF CANCER, vol. 95, no. 1, 20 January 2001 (2001-01-20), pages 67-72, XP002383041 ISSN: 0020-7136
- DEBIES M T ET AL: "Genetic basis of human breast cancer metastasis." JOURNAL OF MAMMARY GLAND BIOLOGY AND NEOPLASIA. OCT 2001, vol. 6, no. 4, October 2001 (2001-10), pages 441-451, XP002383042 ISSN: 1083-3021
- CALLAHAN R ET AL: "NOTCH SIGNALING IN MAMMARY GLAND TUMORIGENESIS" JOURNAL OF MAMMARY GLAND BIOLOGY AND NEOPLASIA, PLENUM PRESS, NEW YORK, NY,, US, vol. 6, no. 1, January 2001 (2001-01), pages 23-36, XP001094918 ISSN: 1083-3021
- LIN W ET AL: "Effects of a water-soluble antitumor ether phosphonoinositide, D-myo-inositol 4-(hexadecyloxy)-3(S)-methoxybutanephospho nate (C4-PI), on inositol lipid metabolism in breast epithelial cancer cell lines." BIOCHEMICAL PHARMACOLOGY. 15 MAY 1999, vol. 57, no. 10, 15 May 1999 (1999-05-15), pages 1153-1158, XP002383043 ISSN: 0006-2952
- NISHIMURA YUKIO ET AL: "Overexpression of ROCK in human breast cancer cells: evidence that ROCK activity mediates intracellular membrane traffic of lysosomes." PATHOLOGY ONCOLOGY RESEARCH : POR. 2003, vol. 9, no. 2, 2003, pages 83-95, XP002383044 ISSN: 1219-4956
- DECRISTOFARO M F ET AL: "Characterization of SWI/SNF protein expression in human breast cancer cell lines and other malignancies." JOURNAL OF CELLULAR PHYSIOLOGY. JAN 2001, vol. 186, no. 1, January 2001 (2001-01), pages 136-145, XP002383045 ISSN: 0021-9541
- SCHWEINFEST CLIFFORD W ET AL: "CaSm: An Sm-like protein that contributes to the transformed state in cancer cells" CANCER RESEARCH, vol. 57, no. 14, 1997, pages 2961-2965, XP002916655 ISSN: 0008-5472
- SAAD Z ET AL: "Expression of genes that contribute to proliferative and metastatic ability in breast cancer resected during various menstrual phases" LANCET THE, LANCET LIMITED. LONDON, GB, vol. 351, no. 9110, 18 April 1998 (1998-04-18), pages 1170-1173, XP004833240 ISSN: 0140-6736
- WAGSTAFF S C ET AL: "Extracellular ATP activates multiple signalling pathways and potentiates growth factor-induced c-fos gene expression in MCF-7 breast cancer cells" CARCINOGENESIS (OXFORD), vol. 21, no. 12, December 2000 (2000-12), pages 2175-2181, XP002383046 ISSN: 0143-3334
- LASTRAIOLI ELENA ET AL: "herg1 gene and HERG1 protein are overexpressed in colorectal cancers and regulate cell invasion of tumor cells." CANCER RESEARCH, vol. 64, no. 2, 15 January 2004 (2004-01-15), pages 606-611, XP002383047 ISSN: 0008-5472
- AL-RAWI M A A ET AL: "Aberrant expression of interleukin-7 (IL-7) and its signalling complex in human breast cancer." EUROPEAN JOURNAL OF CANCER, vol. 40, no. 4, March 2004 (2004-03), pages 494-502, XP002383048 ISSN: 0959-8049
- SUAREZ-CUERVO CATALINA ET AL: "Tumor necrosis factor-alpha induces interleukin-6 production via extracellular-regulated kinase 1 activation in breast cancer cells." BREAST CANCER RESEARCH AND TREATMENT, vol. 80, no. 1, July 2003 (2003-07), pages 71-78, XP002383054 ISSN: 0167-6806
- SHEN TIANJIE ET AL: "Transcriptional hyperactivity of human progesterone receptors is coupled to their ligand-dependent down-regulation by mitogen-activated protein kinase-dependent phosphorylation of serine 294" MOLECULAR AND CELLULAR BIOLOGY, vol. 21, no. 18, September 2001 (2001-09), pages 6122-6131, XP002383055 ISSN: 0270-7306
- BJORNLAND KRISTIN ET AL: "Polymorphonuclear elastase in human colorectal carcinoma" INTERNATIONAL JOURNAL OF ONCOLOGY, vol. 12, no. 3, March 1998 (1998-03), pages 535-540, XP008064807 ISSN: 1019-6439
- MURPHY KELLEY: "Ubiquitin localization within cells in normal and cancer tissues." FASEB JOURNAL, vol. 17, no. 4-5, March 2003 (2003-03), pages Abstract No. 723.6 URL-http://ww, XP008064812 & FASEB MEETING ON EXPERIMENTAL BIOLOGY: TRANSLATING THE GENOME; SAN DIEGO, CA, USA; APRIL 11-15, 2003 ISSN: 0892-6638
- MOODY TERRY W ET AL: "VIP receptor antagonists inhibit mammary carcinogenesis in C3(1)SV40T antigen mice." LIFE SCIENCES, vol. 74, no. 11, 30 January 2004 (2004-01-30), pages 1345-1357, XP002383058 ISSN: 0024-3205

## Description

### Field of the Invention

The present invention relates to a method and kit, including parts thereof, for the prognosis of breast cancer. In particular, the method involves identifying a gene expression pattern that indicates the likelihood of survival of a patient with breast cancer and/or likelihood of recurrence of the disease and/or the metastatic character of the cancer In a patient being treated, or having been treated, for breast cancer.

### Background of the Invention

Breast cancer is the most common female cancer in the UK, US and Denmark. It Is also the most common form of cancer affecting women in the industrialised world. The incidence of breastcancer has been gradually increasing, and in the US, it is the second most common cause of death due to cancer. Indeed, in 1997, it was estimated that 181,000 new cases were reported in the US, and it has been estimated that 40,000 people die of breast cancer every year. Despite the global efforts that have been made to combat this condition, there has been very little change in the incidence of breast cancer, although early detection and new therapies have marginally improved survival over the past few decades.

While the mechanism of tumorigenesis for most breast carcinomas is largely unknown, there are a number of factors thatcan predispose some women to developing breast cancer. These include history of birth, menstrual condition, tumour grade, ER status, the size of tumour and the involvement of lymph nodes at the time of diagnosis and surgery. Additionally, prognosis may be determined to varying degrees by the use of mammography or other x-ray imaging methods. However, a mammogram is not without risk and the breast tumour may be induced by the ionising properties of the radiation used during the test. In addition, such processes are expensive and the results may be interpreted differently by different technicians. For example, one study showed major clinical disagreements in about one third of a set of mammograms that were interpreted by a group of radiologists. Moreover, many women find that undergoing a mammogram is a painful experience.

In clinical practice the prognosis of the disease is important because it determines the treatment that will be given. Accurate prognosis could allow the oncologist to, for example, favour the administration of hormone therapy or chemotherapy and recommend surgery only in the most aggressive cases of cancer.

However, early diagnosis has become a regular feature in breast cancer because more and more patients are now presenting with the disease at a very early stage. This has made conventional methods of assessing the outcome of the cancer more difficult, and it has become increasingly more evident that it is not only the type of cancer but also the timing of the treatment that is key to how well, or poorly, a patient responds. For example, many patients may currently receive unnecessary treatment that frequently causes toxic side effects, whereas other patients may be put on conservative treatment strategies when in fact a cancer is more advanced than predicated, It can therefore be of vital importance that a correct, and accurate, prognosis is made at an early stage.

To date, no set of satisfactory predictors for prognosis based on clinical information alone has been identified. As a result, research has turned to looking at molecular signatures than can diagnose and prognose cancer. WO 02/103320 discloses thousands of genetic markers whose expression is correlated with clinical prognosis, and which can be used to distinguish patients having good prognoses from poorprognoses. The method for determining expression involves comparing the expression pattern of a test sample of tissue taken from a patient with that of a sample of tissue taken from a patient with a good prognosis and also with that of a sample taken from a patient with a known poor prognosis, and determining which of these samples the test sample most closely corresponds to. WO 03/083141 relates to the correlation of the molecular signature of one or more cells of a cytological specimen with the phenotype of one or more cells of a histological sample. Such methods of correlating is accomplished by comparing the molecular signature of the cells of a cytological specimen with the molecular signature of cells corresponding to a particular phenotype. Equivalence between the two signatures Indicate that the cell(s) of the specimen have the phenotype of the sample. In particular WO 03/083141 provides for comparisons of molecular signatures of cytological specimens with "reference" histological signatures of different subtypes of benign conditions as well as various subtypes of malignant conditions of breast cancer. WO 03/083141 furthermore provides for comparisons of molecular signatures of cytological specimens with histological signatures of disease prognosis or outcome phenotypes at the cell, tissue, system, and/or organism level as observed in subjects with cells having the signature in a histological sample. This includes mortality rates, life expectancy under various conditions, sensitivity or resistance to a particular therapeutic agent or treatment.

Although this methodology represents an improvement over the traditional clinical methods of prognosis, it does have a number of drawbacks. For example, analysing hundreds of gene markers takes considerable time and is not inherently practical. Furthermore, it is not clear whether a sample that expresses some of the good prognosis markers and some of the bad prognosis markers would give a prognosis of one or the other option. Accordingly, due to the complexity of the methodology, it may either be inaccurate, in the sense that patients are put in the wrong prognosis group because they express more of the genes in one group than the other, or it may be unable to provide a definite answer. As explained previously, early, and accurate, prognosis is vital for appropriate and effective treatment It is therefore clear that a simpler, more definitive molecular signature is required.

We have therefore developed a method for determining the prognosis of a given breast cancer which is relatively straightforward to perform, efficient to undertake and provides an accurate indication of the likely outcome of the disease. Our method uses a small but highly representative sample of markers which are therefore particularly accurate In determining the likely outcome of a given cancer. Moreover, our method can be divided into three components: a first component that predicts the likely survival of an individual presenting with breast cancer, a second component that predicts the likely recurrence of cancer in an individual presenting with breast cancer; and a third component that predicts the metastatic character of the cancer. As will be apparent to those skilled in the art, the second component therefore indicates the likelihood of incidence free survival of a patient presenting with breast cancer and the third component indicates the aggressive nature of the disease. In summary, we have identified a plurality of molecular signatures that have relevance in determining the prognosis of a given breast cancer. Each molecular signature comprises a plurality of genetic markers whose expression, either high or low in respect of tissue from a patient with moderate prognosis (see hereinafter), is indicative of a given outcome. In addition to this, we have analysed each molecular signature in order to identify which genetic markers are the best indicators of the outcome of a given disease, in other words those that contribute most to the predictive ability of the molecular signature. This subset of markers is known, collectively, as the refined molecular signature.

For example, there is provided a first molecular signature, which comprises two sets of molecular markers whose high expression correlates with low survival rate; the first set comprises those molecular markers that are the most statistically significant indicators of low survival rate, these are referred to herein, collectively, as the first primary molecular signature [Set (A)]:
AMF, ATF4. Cyr61, ER, Matriptase2, MET, MLN64, MMP7, Nectin4, PAR1A, Psoriason. Pttg1, Rho-C, Scotin, SDF1. SEMP1, SPF45, SST1. ST15, TACC2. TBD10. TCF2. TEM6. TEM7R, ZO-3: and
the second set comprises the afore plus at least one of the following molecular markers, referred to herein, collectively, as the first secondary molecular signature [Set (B)]:
Basigin, Beta-catenin, BMP1, BMP10. Calpain large, CD44, CX43, cyclinD2. EHMS, FAK, FAP, GIRK, HAVR1, Isotopo3, JAK1, LOX12, NET-2, PAR1A2, PTHrP, Rho-G, S100A4, SPARC, TCF3, VECAD, Vilip, Wave2.

Reference herein to the above, and following, markers is reference to a named protein whose full identity is available on the www.NCBI.LM.NIH.gov database or is well known to those skilled in the art.

Reference herein to high or low expression is with respect to the level of expression of the same marker in patients who were deemed to have a moderate prognosis i.e. patients with a standard prognostic index Nottingham Prognosis Index (NPI) = 3.4-5.4, where the NPI = 0.2 x tumour size + tumour grade + nodal status where
NPI (low) is <3.4 and 86% of patients survive 15 years
NPI (moderate) is 3.4 - 5.4 and 42% of patients survive 15 years
NPI (high) is >5.4 and 13% of patients survive 15 years.

There is further provided a second molecular signature, which comprises two sets of molecular markers whose low expression correlates with low survival rate; the first set comprises those molecular markers that are the most statistically significant indicators of low survival rate, these are referred to herein, collectively, as the second primary molecular signature [Set (C)]:
ARP2, Atf-3, HuR, MEN1, Paracellin, PTP-RK Radixin, RHO8/gdiG-Ratio; and
the second set comprises the afore plus at least one of the following molecular markers, referred to herein, collectively, as the second secondary molecular signature [Set (D)]:
   aMOT, Atf-1, Claudln-1. IL22R, Rock 2 Veg1.

There is yet further provided a third molecular signature, which comprises two sets of molecular markers whose high expression correlates with a low incidence of cancer free survival; the first set comprises those molecular markers that are the most statistically significant indicators of a low incidence of cancer free survival, these are referred to herein, collectively, as the third primary molecular signature [Set (E)]:
AAMP, AMFR, Bmp8, BMP9, Betacatenin, CAR, Creb12, DRIM, EHMS, Endomuscin2, FAK, FAP, isotopo1, Kiss1/dck19, Notch1, PAR1A, Par1A2, PLC-delta, Psoriasin, PTTG1, RhoC, Rock1, SDF1, SST1, ST15, TEM6, TEM7R; and
the second set comprises the afore plus at least one of the following molecular markers, referred to herein, collectively, as the third secondary molecular signature [Set (F)]:
   Angiotensin2R1, ATF4, Bmp10, CASM, cathepsinS, CX43, Elastase PMN, GIRK, HAVR1. HIN, Isotopo3, Kiss1, LOX12, NOS3, PMSA, S100A4, SEMP1, TACC2. Ubiquitin, WISP2.

There is further provided a fourth molecular signature, which comprises two sets of molecular markers whose low expression correlates with a low incidence of cancer free survival; the first set comprises those molecular markers that are the most statistically significant indicators of a low incidence of cancer free survival, these are referred to herein, collectively, as the fourth primary molecular signature [Set (G)]:
Bmp3, IL22R. IL24, JAK1, PTP-RK, Rho8/GdiG, Snail, WASP; and
the second set comprises the afore plus at least one of the following molecular markers, referred to herein, collectively, as the fourth secondary molecular signature [Set (H)]:
   ATF3, Bmp4, BMPR1A. MEN1. Paracellin.

There is yet further provided a fifth molecular signature which comprises two sets of molecular markers whose high expression correlates with metastatic cancer; the first set comprises those molecular markers that are the most statistically significant indicators of a metastatic cancer, these are referred to herein, collectively, as the fifth primary molecular signature [Set (I)]:
BAF57, BNDF, CAR1. CASM, Cathepsin-L, Creb1/2. CXCR10, DRIM, HERG, IL7R, IL-11, Kiss1, MKK1, PMN-elastase, PTTP1, SDF5, TACC2, Ubiquitin, VIPR1, VUDP; and
the second set comprises the afore plus at least one of the following molecular markers referred to herein, collectively, as the fifth secondary molecular signature [Set (J)]:
   Anglomotin, BMP7. cyclinD1, DNA ligase-1, IGFBP7, LYVE1, NET2, RHO8, SRBC, Stath4, TGAse-3, Vinculin, WAVE2.

Finally, there is provided a sixth molecular signature which comprises two sets of molecular markers whose low expression correlates with metastatic cancer; the first set comprises those molecular markers that are the most statistically significant Indicators of a metastatic cancer, these are referred to herein, collectively, as the sixth primary molecular signature [Set (K)]:
Paracellin; and
the second set comprises the afore plus at least one of the following molecular markers referred to herein, collectively, as the sixth secondary molecular signature [Set (L)]:
   ALCAM, Eplin, ERbeta, Glyplc3, JAK1, MAGI-1, PEDF, PKC-eta, Stathlin, WWOX.

We have therefore determined at least six molecular signatures, comprising twelve sets of molecular markers (six primary and six secondary), which have use in the prognosis of breast cancer. The elucidation of these signatures has involved over a decade of work during which time we have systematically and carefully examined hundreds of samples of breast cancer tissue and many more hundreds of genetic molecular markers. However, having completed this arduous task we have, surprisingly, found that, in fact, very few genes need to be examined in order to provide an accurate prognosis for a given sample of breast cancer tissue. Even more surprisingly, we have been able to further reduce this number by identifying those molecular markers that contribute most to the predictive outcome of our molecular signatures, so for example, in the case of the molecular signature relating to metastatic cancer, only 20/21 genes need to be examined. This means that our methodology has immediate application and could be performed quickly and routinely in a clinical context. In fact, we suggest that our methodology forms part of the standard treatment regime of a breast cancer patient so that the relevant oncologist can, at an early stage, determine the outcome of a particular disease and so match the treatment accordingly. Thus, for example, in the case of an individual who presents with a signature indicative of low survival or node metastasis (i.e. the cancer Is likely to spread) an immediate and aggressive form of therapy might be prescribed. Similarly, where an individual presents with a signature indicative of low disease-free survival, and therefore is more likely to have a recurrence of the disease, more frequent follow-up visits and tests might be required. Conversely, it an individual has a signature indicative of no metastasis, the oncologist can prescribe less invasive and aggressive treatment, thereby saving the patient from any unnecessary distress and unwanted side effects. Our method therefore not only serves to ensure that individuals receive treatment tailored to their genetic make-up, but it can improve the quality of a patient's life during treatment, by ensuring that aggressive therapy is only prescribed in those cases where it is necessary.

Accordingly, in one aspect of the invention there is provided a method for determining the prognosis of mammalian breast cancer, which method comprises:
(a) examining a sample of breast cancer tissue from an Individual in order to determine the expression level of genes encoding the molecular markers in Set (A) and Set (C), and;
(b) where a high level of expression is determined for the Set (A) markers and a low level of expression is determined for the Set (C) markers;
(c) concluding that the Individual from whom the tissue sample has been taken has a low likelihood of survival, that is less than 20% of the individuals survive more than 5 years.

In yet a further preferred embodiment of the invention said methodology, In part (a) thereof, additionally comprises determining the expression level of genes encoding the molecular marker in Set (B), in order to determine whether these genes have a high level of expression; and determining the expression level of genes encoding the molecular markers in Set (D), in order to determine whether these genes are under expressed and, if the above expression patterns are identified, concluding that the individual has a low likelihood of survival that is less than 20% of the individuals survive more than 5 years.

In yet a further aspect of the invention there is provided a method for determining the prognosis of mammalian breast cancer, which method comprises: ,
(a) examining a sample of breast cancer tissue from an individual in order to determine the expression level of genes encoding the molecular markers in Set (C), and;
(b) where a low level of expression is determined for these markers;
(c) concluding that the individual from whom the tissue sample has been taken has a low likelihood of survival.

In yet a further preferred embodiment of the invention, said methodology, in part (a) thereof, additionally, or alternatively, comprises determining the expression level of genes encoding at least one molecular marker in Set (D) in order to determine whether these genes are under expressed and, if they are, concluding that the individual has a low likelihood of survival.

In yet a further preferred embodiment of this aspect of the invention said methodology, in part (a) thereof, additionally comprises determining the expression level of genes in Set (A) and/or at least one gene in Set (B) in order to determine if these genes are over expressed and, if they are, concluding that the individual has a low likelihood of survival.

In yet a further aspect of the invention there is provided a method for determining the prognosis of mammalian breast cancer, which method comprises:
(a) examining a sample of breast cancer tissue from an individual in order to determine the expression level of genes encoding the molecular markers in Set (E), and;
(b) where a high level of expression is determined for these markers;
(c) concluding that the individual from whom the tissue sample has been taken has a high likelihood of cancer recurrence.

Reference herein to cancer recurrence includes reference to the recurrence of cancer locally, in the breast, or at a remote site or reference to metastasis.

In yet a further preferred embodiment of the invention, the methodology additionally comprises, in part (a) thereof, determining the expression level of genes encoding at least one molecular marker in Set (F), in orderto determine whether these genes have a high level of expression; and/or determining the expression level of genes encoding the molecular markers in Set (G), in order to determine whether these genes are under expressed; and/or determining the expression level of genes encoding at least one molecular marker in Set (H), in order to determine whether these genes are under expressed, and if the above expression patterns are identified, concluding that the individual has a high likelihood of cancer recurrence.

In yet a further aspect of the invention there is provided a method for determining the prognosis of mammalian breast cancer, which method comprises:
(a) examining a sample of breast cancer tissue from an individual in order to determine the expression level of genes encoding the molecular markers in Set (G), and;
(b) where a low level of expression is determined for these markers;
(c) concluding that the individuals from whom the tissue sample had been taken has a high likelihood of cancer recurrence.

In yet a further preferred embodiment of the invention, said methodology, in part (a) thereof, additionally, or alternatively, comprises determining the expression level of genes encoding at least one molecular marker in Set (H), in order to determine whether these genes are under expressed and, if they are, concluding that the individual has a high likelihood of cancer recurrence.

In yet a further preferred embodiment of this aspect of the invention said methodology, in part (a) thereof, additionally comprises determining the expression level of genes in Set (E) and/or at least one gene in Set (F) in order to determine if these genes are over expressed and, if they are, concluding that the individual has a high likelihood of cancer recurrence.

In yet a further aspect of the invention there is provided a method for determining the prognosis of mammalian breast cancer, which method comprises:
(a) examining a sample of breast cancer tissue from an individual In order to determine the expression level of genes encoding the molecular markers in Set (I), and;
(b) where a high level of expression is determined for these markers;
(c) concluding that the individual from whom the tissue sample has been taken has a metastatic form of cancer.

In yet a further preferred embodiment of the invention, the methodology additionally comprises, in part (a) thereof, determining the expression level of genes encoding at least one molecular markerin Set (J), in orderto determine whether these genes have a high level of expression; and/or determining the expression level of the gene encoding the molecularmarkerin Set (K), in orderto determine whether this gene is underexpressed; and/or determining the expression level of genes encoding at least one molecular marker in Set (L), in order to determine whether these genes are under expressed and, if the above expression patterns are Identified, concluding that the individual has a metastatic form of cancer.

in yet a further aspect of the invention there is provided a method for determining the prognosis of mammalian breast cancer, which method comprises:
(a) examining a sample of breast cancertissue from an individual in orderto determine the expression level of genes encoding the molecular marker in Set (K), and;
(b) where a low level of expression is determined for this marker,
(c) concluding that the individual from whom the tissue sample has been taken has a metastatic form of cancer.

In yet a further preferred embodiment of the invention, said methodology, in part (a) thereof, additionally, or alternatively, comprises determining the expression level of genes encoding at least one molecular marker in Set (L) in order to determine whether these genes are under expressed and, if they are, concluding that the individual has a metastatic form of cancer.

In yet a further preferred embodiment of this aspect of the invention said methodology, In part (a) thereof, additionally comprises determining the expression level of genes in Set (I) and/or at least one molecular marker in Set (J) in orderto determine if these genes are over expressed and, if they are, concluding that the individual has a metastatic form of cancer.

In a further aspect of the invention there Is provided any selected combination of all the aforementioned method.

In each of the above methods of the invention, the assay is, ideally, undertaken for human breast cancer tissue and, more preferably still, fernale human breast cancer tissue.

In each of the above methods of the invention, ideally, the sample of tissue that is examined is assayed for the presence of RNA, preferably total RNA and, more preferably still, the amount of mRNA. It will be apparent to those skilled in the art that techniques available for measuring RNA content are well known and, indeed, routinely practised by those in the clinical diagnostics field.

In an alternative embodiment of the invention the method involves assaying for the protein encoded by each of the molecular markers and so, typically, but not exclusively, involves the use of agents that bind to the relevant proteins and so identify same. Common agents are antibodies and, most ideally, monoclonal antibodies which, advantageously, have been labelled with a suitable tag whereby the existence of the bound antibody can be determined. Assay techniques for identifying proteins are well known to those skilled in the art and indeed used every day by workers in the field of clinical diagnostics.

Additionally, the methodology of the invention may involve the amplification of a selected marker prior to the identification of same and in this case, typically, amplification will be undertaken using a PCR reaction wherein oligonucleotide probes specific for the molecular marker of Interest are used in order to amplify same prior to determining the presence and, having regard to the degree of amplification, the amount thereof.

In further preferred methods of working the invention the level of expression of a given molecular marker is determined having regard to a control sample, wherein the control sample is a sample of breast tissue which is cancer free or from a patient with a moderate prognosis as hereindefined. More ideally still this sample of breast tissue b taken from an individual who is not presenting with the disease. Alternatively still, the control is a recognised standard for expression of each relevant gene In a healthy individual.

The level of gene expression may be measured by real-time quantitative PCR, using a method disclosed in Jiang et al 2003a or Parr and Jiang 2004.

The likelihood of survival means the likelihood that the patient will be alive for the next 10 years. The likelihood of recurrence means the likelihood that the cancer will recur within 10 years. A metastatic form of cancer means that the cancer will have spread from the organ or tissue of origin to another part of the body.

According to yet a further aspect of the invention there is provided a kit for performing any one or more of the aforementioned methods wherein said kit comprises:
(a) a plurality of probes for detecting at Wast one Set of the molecular markers specified in the aforementioned methods; and
(b) optionally, reagents and instructions pertaining to the use of said probes.

In yet a further preferred aspect of the invention there is provided a kit for determining the prognosis of mammalian breast cancer which comprises:
(a) a plurality of probes for identifying at least one transcript of each of the genes in Set (A) and set (c), and:
(b) optionally, reagents and instructions that determine, or show how to determine, the level of expression of each of said genes.

In yet a further embodiment of the invention, said kit additionally comprises:
(a) a plurality of probes for identifying: at least one transcript for the genes In Set (B) or (D), and;
(b) optionally, reagents and instructions that determine, or show how to determine, the level of expression of each of said genes.

In yet a further preferred aspect of the invention there Is provided a kit for determining the prognosis of mammalian breast cancer which comprises:
(a) a plurality of probes for identifying at least one transcript of each of the genes in Set (E), and;
(b) optional, reagents and instruction that determine, or show to determine, the level expression of each of said genes.

In yet a further preferred aspect of the invention said kit additionally comprises:
(a) a plurality of probes for identifying: at least one transcript of each of the genes in Set (G) and/or at least one transcript for at least one of the genes in Set (F),or (H), and;
(b) optionally, reagents and instructions that determine, or show how to determine, the level of expression of each of said genes.

In yet a further preferred aspect of the invention there is provided a kit for determining the prognosis of mammalian breast cancer which comprises:
(a) a plurality of probes for identifying at least one transcript of each of the genes in Set (I), and;
(b) optionally, reagents and instructions that determine, or show how to determine, the level of expression of each of said genes.

In yet a further preferred aspect of the invention said kit additionally comprises:
(a) a plurality of probes for identifying: at least one transcript of each of the genes in Set (K), and/or at least one transcript for at least one of the genes in Set (J) or (L), and:
(b) optionally, reagents and instructions that determine, or show how to determine, the level of expression of each of said genes.

in a further aspect of the invention there is provided a kit comprising any selected combination of the aforementioned sets of probes for identifying the aforementioned sets of molecular markers.

According to yet further aspect of the invention there Is provided a microarray comprising any one or more of the aforementioned sets of probes for identifying the level of expression of any one or more of the aforementioned sets of molecular markers.

In another aspect of the invention, there is provided a kit for determining the likelihood of survival and/or recurrence of breast cancer and/or metastatic nature of a cancer in a patient, which kit comprises:
(a) at least one microarray comprising a plurality of probes for identifying at least one set of the molecular markers described in the above methods; and, optionally,
(b) a second microarray comprising a plurality of probes for identifying the same set of molecular markers in an internal standard that represents the norman level of expression of said markers.

The invention also provides a microarray or set of probes as described above.

The present invention will now be described by way of the following examples with reference to Tables 1-3 and Figures 1-4 wherein:
Figure 1 shows a Kaplan-Meler survival curve for all the markers in Table 1.
Figure 2 shows a Kaplan-Meler survival curve for markers indicated with a in Table 1.
Figure 3 shows a Kaplan-Meler survival curve for all the markers in Table 2.
Figure 4 shows a Kaplan-Meler survival curve for markers indicated with a in Table 2.

### Tissues and cells

Breast tumour tissues and associated normal tissues were collected immediately after surgery and frozen until use. This was under the approval of a local ethical committee and took place mainly between 1991-1994, with limited number collected between 1995-1996. The current analysis is based on a median follow up of 10 years as at June 2004. The current study has used breast cancer tissues (n=120) and normal background tissues (n=32). Human breast cancer cell lines MCF-7 and MDA MB 231, human fibroblast cell line MRC-5 were purchased from the European Collection of Animal Cell Cultures (ECACC, Salisbury, England). Human umbilical vein endothelial cells (HUVEC) were purchased from TCS Biologicals (Oxford, England). Information on the pathology, clinical information during and after surgery, patient clinical outcomes were obtained soon after surgery or at the time of follow up.

### Tissues processing

Mammary tissues were frozen sectioned. Sections were divided into the following three parts: one portion for routine histology, one portion for immunohistochemistry and the other portion was for preparation of RNA.

### Extraction of RNA from cells and tissues and cONA synthesis

Frozen sections of tissues were cut at a thickness of 5-10µm and were kept for immunohistochemistry and routine histology (Jiang *et al* 2003a). A further 15-20 sections were homogenised using a hand-held homogeniser, In ice-cold RNA extraction solution. The concentration of RNA was determined using a UV spectrophotometer. Reverse transcription was carried using a RT kilt with an anchored cligo-dt primer supplied by AbGene™, using 1 µg total RNA in 96-well plate. The quality of cDNA was verified using β-actin primers. RNA extraction kit and RT kit were obtained from AbGene Ltd, Surrey, England, UK. PCR primers were designed using Beacon Designer (California, USA) and synthesised by Invitrogen™ Ltd (Paisley, Scotland, UK). Molecular biology grade agarose and DNA ladder were from Invitrogen. Mastermix for routine PCR and quantitative PCR was from AbGene.

### Quantitative analysis of genetic markers

The transcript level of the CCN family members from the above-prepared cDNA was determined using a real-time quantitative PCR, based on the Amplifuor™ technology (Nazarenko *et al* 1997), modified from a method previous reported (Jiang *et al* 2003a and 2003b). Briefly, a pair of PC R primers were designed using the Beacon Designer software (version 2, California, USA). To one of the primers (routinely to the antisense primer in our laboratory), an additional sequence, known as the Z sequence (5' actgaacctgaccgtaca'3) which is complementary to the universal Z probe (Nazarenko et at 1997) (Intergen Inc., England, UK), was added. A Taqman™ detection kit for β-actin was purchased from Perkin-Elmer™.

The reaction was carried out using the following: Hot-start Q-master mix (Abgene), 10pmol of specific forward primer, 1pmol reverse primer which has the Z sequence, 10pmol of FAM-tagged probe (Intergen Inc.), and cDNA from approximate 50ng RNA (calculated from the starting RNA in the RT reaction). The reaction was carried out using lcyclerlQ™ (Bio-Rad™) which is equipped with an optic unit that allows real time detection of 96 reactions, using the following condition: 94°C for 12 minutes, 50 cycles of 94°C for 15 seconds, 55°C for 40 seconds and 72°C for 20 seconds (Jiang et al 2003b, 2003c, Parr and Jiang 2004). The levels of the transcripts were generated from an internal standard (Jiang et al 2003a) that was simultaneously amplified with the samples. The results are shown here in two ways: levels of transcripts based on equal amounts of RNA, or as a target/CK19 ratio.

### Immunohistochemical staining of the molecule where appropriate

Frozen sections of breast tumour and background tissue were cut at a thickness of 6µm using a cryostat (Jiang *et al* 2003c). The sections were mounted on super frost plus microscope slides, air dried and then fixed in a mixture of 50% acetone and 50% methanol. The sections were then placed in "Optimax" wash buffer for 5-10 minutes to rehydrate. Sections were incubated for 20 mins in a 0.6% BSA blocking solution and probed with a primary antibody. Following extensive washings, sections were incubated for 30 minutes in a secondary biotinylated antibody (Multilink Swine anti-goat/mouse/rabbit immunoglobulin, Dako Inc.). Following washings, Avidin Biotin Complex (Vector Laboratories) was then applied to the sections followed by extensive washings. Diaminobenzidine chromogen (Vector Labs) was then added to the sections which were incubated in the dark for 5 minutes. Sections were then counter stained In Gill's Haematoxylin and dehydrated in ascending grades of methanol before clearing in xylene and mounting under a cover slip. Cytoplasmic staining of the respective proteins was quantified using Optimas 6.0 software as we previously described (Davies et al 2000, King et al 2004) and is shown here as relative staining intensity.

### Statistical analysis

Statistical analysis was carried out using Mann-Whitney U test and the Kruskel-Wallis test. Survival analysis was carried out using Kaplan-Meier survival curve and Univariate analysis (SPSS11).

### RESULTS

### Molecules screened

We have quantified 453 molecules against the full clinical information that includes a 10-year follow up. Following analysis on survival rates and incidence of recurrence of the disease, we have developed three signatures, the survival molecular signature and the incidence prediction molecular signature and the metastatic molecular signature.

### The survival molecular signature

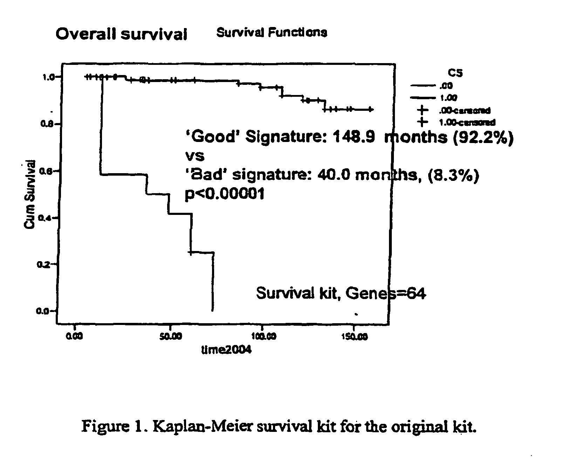

As shown in Table 1,51 molecules were found to have a positive correlation with low survival and 14 inversely correlated with low survival. Figure 1 shows that 92.2% of individuals having what is termed herein as a "good signature" (that Is not having a high expression of the molecules in the left-hand column of Table 1, and not having under expression of the molecules in the right-hand column of Table 1), are predicted to survive for up to 148.9 months, while only 8.3% of individuals having what is termed herein as a "bad signature" (that is having a high expression of the molecules in the left-hand column of Table 1, and under expression of the molecules in the right-hand column of Table 1) are predicted to survive for up to 40 months. This result is statistically significant, with a p value < 0.00001.

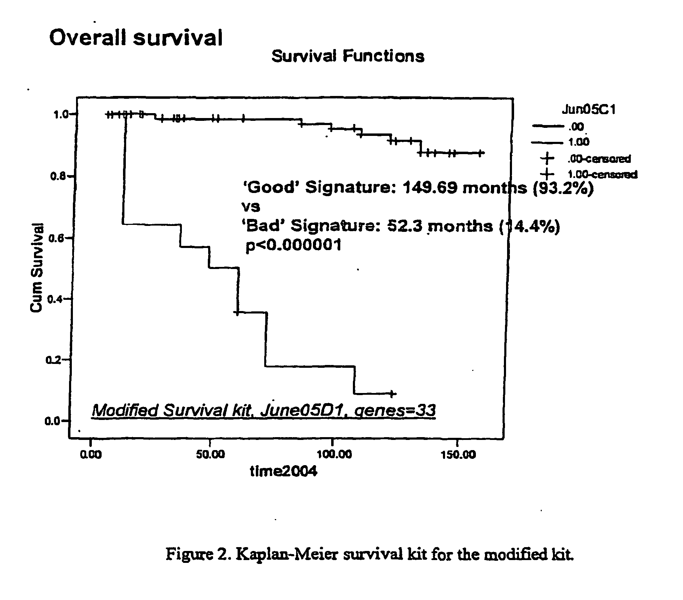

Using the Kaplan-Meier survival curve and univariate analysis, we refined the molecular signature by identifying those molecules that contribute most to the statistical accuracy (identified by in Table 1). We have found that 33 primary molecular markers, 25 of which have a positive correlation with low survival and 8 of which have a negative correlation with low survival, account for the majority of the statistical significance. Figure 2 shows the predicted survival curve using the first primary and second primary molecular signature, with 93.2% of individuals having a good signature predicted to survive for up to 149.69 months, and only 14.4% of individuals having a bad signature predicted to survive for up to 52.3 months (p < 0.000001).

### The incidence free prediction molecular signature

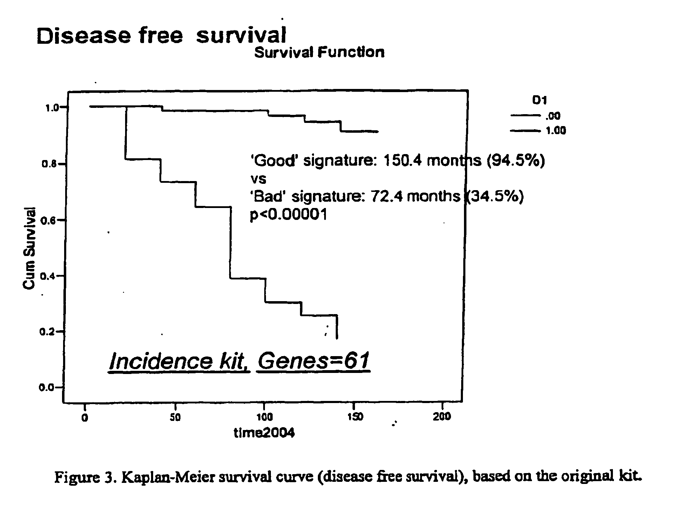

As shown in Table 2, 48 molecules were found to have a positive correlation with occurrence of incidence (recurrence and metastasis) and 13 inversely correlated with occurence of incidence. Figure 3 shows that 94.5% of individuals having a good signature (that is not having a high expression of the molecules in the left-hand column of Table 2, and not having under expression of the molecules in the right-hand column of Table 2) are predicted to have no recurrence of the disease (i.e. disease -free survival) for up to 150.4 months, while only 34.5% of individuals having a bad signature (that is having a high expression of the molecules in the left-hand column of Table2, and under expression of the molecules in the right-hand column of Table 2) are predicted to live disease-free for only up to 72.4 months (p < 0.00001).

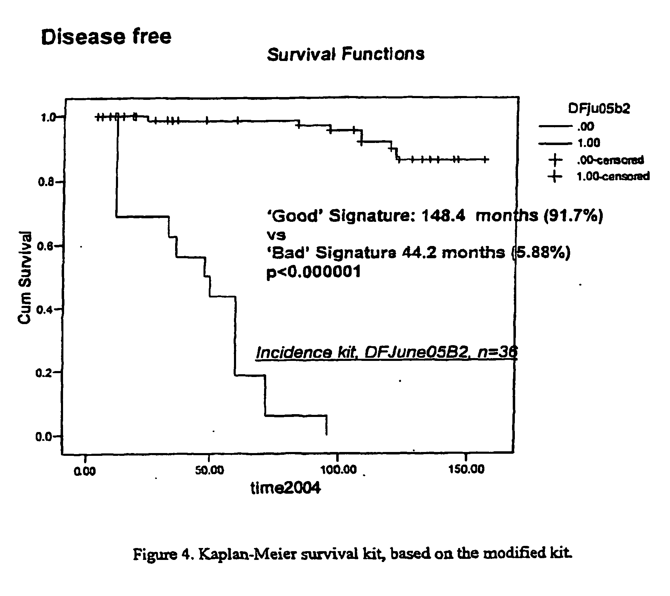

As with the survival signature above, we also refined this signature, and found that 36 primary molecular markers (indicated by in Table 2), 28 of which have a positive correlation with recurrence, and 8 of which have a negative correlation with recurrence account for the majority of the statistical significance. Figure 4 shows the predicted survival curve using the third primary and fourth primary molecular signature, with 91.7% of individuals having a good signature predicted to have no recurrence of the disease for up to 148.4 months, and only 5.88% of individuals having a bad signature predicted to survive, without any recurrence of the disease, for up to 44.2 months (p < 0.000001).

### The molecular signature of node metastasis

As shown in Table 3, 37 molecules were found to have a positive correlation with nodal metastasis and 10 inversely correlated with node metastasis. The combination of these 37 molecules has shown that 91 % of tumours with a bad signature (that is having a high expression of the molecules in the left-hand column of Table 3, and under expression of the molecules in the right-hand column of Table 3) developed node metastasis. Furthermore, 88.9% of tumours with a good signature (that is not having a high expression of the molecules in the left-hand column of Table 3, and not having under expression of the molecules in the right-hand column of Table 3) had no node metastasis (p = 0.00024).

As above, we have modified this signature by refining the combination, and found that a combination of 21 primary genes, 20 of which are positively correlated with node metastasis and 1 of which is negatively correlated with node metastasis (indicated by in Table 3), also predicted well. 89.1% of tumours with a bad signature had node metastasis and 86.8% of tumours with a good signature had no node metastasis (p = 0.0000205).

### References

Davies at al 2000: Davies G, Jiang WG, Mason MD. Cell-cell adhesion and signalling intermediates in human prostate cancer. Journal of Urology, 2000, 163, 985-992
Jiang et al 2003a: Jiang WG, Watkins G, Lane J, Douglas-Jones A, Cunnick GH, Mokbel M, Mansel RE. Prognostic value of Rho family and and rho-GDIs in breast cancer. Clinical Cancer Research, 2003, 9 (17), 6432-6440
Jiang et al 2003b: Jiang WG , Douglas-Jones A, and Mansel RE. Level of expression of PPAR-gamma and its coactivator (PPAR-GCA) in human breast cancer. International Journal of Cancer, 2003, 106, 752-757
Jiang et al 2003c: Jiang WG, Grimshaw D, Lane J, Martin TA, Parr C, Davies G, Laterra J, and Mansel RE. Retroviral hammerhead transgenes to cMET and HGF/SF inhibited growth of breast tumour, induced by fibroblasts. Clinical Cancer Research, 2003, 9, 4274-4281
King et al 2004: King JAC, Ofori-Acquah AF, Stevens T, Al-Mehdi AB, Fodstad O, Jiang WG. Prognostic value of ALCAM In human breast cancer. Breast Cancer Research, 2004, R478-487
Nazarenko et al 1997: Nazarenko IA, Bhatnagar SK, Hohman RJ. A closed tube format for amplification and detection of DNA based on energy transfer. Nucleic Acids Res 1997;25: 2516-21
Parr and Jiang 2004: Parr C and Jiang WG. The Notch receptors, Notch-1 and Notch-2, in human breast cancers. International Journal of Molecular Medicine, 2004 Nov;14(5): 779-786

**Table 1. Molecular signature for overall survival**

| **High with incidence** | | **Low with incidence** | |
|---|---|---|---|
| Original kit (survival curve is figure 1) | Modified kit (genes (indicated by*, survival curve is figure 2) | Original kit (figure 1) | Modified kit (genes (indicated by*, figure 2) |
| AMF | * | aMOT | |
| ATF4 | * | ARP2 | * |
| Basigin | | Atf-1 | |
| Beta-catenin | | Atf-3 | * |
| BMP1 | | Claudin-1 | |
| BMP10 | | HuR (0.05) | * |
| Calpain large | | IL22R | |
| CD44 | | MEN1 (0.02) | * |
| CX43 | | Paracellin | * |
| cyclinD2 | | PTP-RK | * |
| Cyr61 | * | Radixin | * |
| EHMS | | RHO8/gdlG-Ratio | * |
| ER | * | Rock 2 | |
| FAK | | VEG1 | |
| FAP | | | |
| GIRK | | | |
| HAVR1 | | | |
| Isotopo3 | | | |
| JAK1 | | | |
| LOX12 | | | |
| Matriptase2 | | | |
| MET | * | | |
| MLN64 | * | | |
| MMP7 | * | | |
| Nectin4 | * | | |
| NET-2 | | | |
| PAR1A | * | | |
| PAR1A2 | | | |
| Psoriason | * | | |
| PTHrP | | | |
| Pttg1 | * | | |
| Rho-C | * | | |
| Rho-G | | | |
| S100A4 | | | |
| Scotin | * | | |
| SDF1 | * | | |
| SEMP1 | * | | |
| SPARC | | | |
| SPF45 | * | | |
| SST1 | * | | |
| ST15 | * | | |
| TACC2 | * | | |
| TBD10 | * | | |
| TCF2 | * | | |
| TCF3 | | | |
| TEM6 | * | | |
| TEM7R | * | | |
| VECAD | | | |
| Vilip | | | |
| Wave2 | | | |
| ZO-3 | * | | |

**Table 2. Molecular signature for incidence free survival In human breast cancer**

| Original kit, genes =61 Modified kit, genes =36 | | | |
|---|---|---|---|
| **High with incidence** | | **Low with incidence** | |
| Original kit (survival curve is figure 3) | Modified kit (genes (indicated by *, figure 4) | Original kit (figure 3) | Modified kit (genes (indicated by *, figure 4) |
| AAMP | * | ATF3 | |
| AMFR | * | Bump3 * | |
| Angiotensin2R1 | | Bmp4 | |
| ATF4 | | BMPR1A | |
| Bmp8 | * | IL22R | |
| BMP9 | * | IL24 | * |
| Bmp10 | | JAK1 | * |
| Beta-catenin | * | MEN1 | * |
| CAR | * | Paracellin | |
| CASM | | PTP-RK | * |
| cathepsinS | | Rho8/GdiG | * |
| Creb12 | * | Snail | * |
| CX43 | | WASP | * |
| DRIM | * | | |
| EHMS | * | | |
| Elastase PMN | | | |
| Endomuscin2 | * | | |
| FAK | * | | |
| FAP | * | | |
| GIRK | | | |
| HAVR1 | | | |
| HIN | | | |
| Isotopo 1 | * | | |
| Isotopo3 | | | |
| Kiss1 | | | |
| Kiss1/ck19 | * | | |
| LOX12 | | | |
| NOS3 | | | |
| Notch1 | * | | |
| PAR1A | * | | |
| Par1A2 | * | | |
| PLC-delta | * | | |
| PMSA | | | |
| Psoriasin | * | | |
| PTTG1 | * | | |
| RhoC | * | | |
| Rock1 | * | | |
| S100A4 | | | |
| SDF1 | * | | |
| SEMP1 | | | |
| SST1 | * | | |
| ST15 | * | | |
| TACC2 | | | |
| TEM6 | * | | |
| TEM7R | * | | |
| Ubiquitin | | | |
| WISP2 | | | |

**Table 3. Molecular signature for predicting node metastasis**

| **Significantly high with node metastasis** | | **Significiantly low with incidence** | |
|---|---|---|---|
| **Initial filing** | **Modified signature(*)** | **Initial filing** | **Modified signature (*)** |
| Anglomotin | | ALCAM | |
| BAF57 | * | Eplin | |
| BMP7 | | ERbeta | |
| BNDF | * | Glypic3 | |
| CAR1 | * | JAK1 | |
| CASM | * | MAGI-1 | |
| Cathepsin-L | * | Paracellin | * |
| Creb1/2 * | | PEDF | |
| CXCR10 * | | PKC-eta | |
| cyclinD1 | | Stathlin | |
| DNA ligase-1 | | WWOX | |
| DRIM | * | | |
| HERG | * | | |
| IGFBP7 | | | |
| IL7R | * | | |
| IL-11 | * | | |
| Kiss1 | * | | |
| LYVE1 | | | |
| MKK1 | * | | |
| NET2 | | | |
| PMN-elastase | * | | |
| PTTP1 | * | | |
| RHO8 | | | |
| SDF5 | * | | |
| SRBC | | | |
| Stath4 | | | |
| TACC2 | * | | |
| TGAse-3 | | | |
| Ubiquitin | * | | |
| Vinculin | | | |
| VIPR1 | * | | |
| VUDP | * | | |
| WAVE2 | | | |

## Claims

1. A method for determining the prognosis of mammalian breast cancer, which method comprises:
(a) examining a sample of breast cancer tissue from an individual in order to determine the expression level of genes encoding the following first set of molecular markers: AMF, ATF4, Cyr61, ER, Matriptase2, MET, MLN64, MMP7, Nectin4, PAR1A, Psoriason, Pttg1, Rho-C, Scotin, SDF1, SEMP1, SPF45, SST1, ST15, TACC2, TBD10, TCF2, TEM6, TEM7R, and ZO-3; and the following second set of molecular markers: ARP2, Atf 3, HuR, MEN1, Paracellin, PTP-RK Radixin, and RHO8/gdiG-Ratio; and
(b) where a high level of expression is determined for the first set of molecular markers and a low level of expression is determined for the second set of molecular markers;
(c) concluding that the individual from whom the tissue sample has been taken has a low likelihood of survival that is less than 20% of individuals survive more than five years.

2. A method according to Claim 1, wherein part (a) additionally comprises determining the expression level of genes encoding the following expanded first set of molecular markers: Basigin, Beta-catenin, BMP1, BMP10, Calpain large, CD44, CX43, cyclinD2, EHMS, FAK, FAP, GIRK, HAVR1, Isotopo3, JAK1, LOX12, NET-2, PAR1A2, PTHrP, Rho-G, S100A4, SPARC, TCF3, VECAD, Vilip, Wave2; and the following expanded second set of molecular markers aMOT, Atf-1, Claudin-1, IL22R, Rock 2, Veg1.

3. A method according to any preceding claim, wherein the cancer tissue is from a human..

4. A method according to any preceding claim, wherein the cancer tissue is from a female.

5. A method according to any preceding claim, wherein the level of expression is determined by assaying for the presence of RNA or mRNA.

6. A method according to any one of Claims 1-5, wherein the level of expression is determined by assaying for the protein(s) encoded by the molecular markers.

7. A method according to Claim 6, wherein the method involves the use of agents that bind to the relevant protein(s) and so identify same.

8. A method according to Claim 7, wherein the agents are antibodies.

9. A method according to any of Claims 1-4, wherein prior to performing part (a), the selected marker is amplified.

10. A method according to Claim 9, wherein the marker is amplified by PCR.

11. A method according to any preceding claim, wherein the level of expression of a given molecular marker is determined having regard to a control sample, wherein the control sample is any one of the following: a sample of breast tissue which is cancer free, a sample of breast tissue taken from an individual who is not presenting with cancer, or a recognised standard for expression of each relevant molecular marker in a healthy individual.

12. A kit for determining the prognosis of mammalian breast cancer which consists of :
(a) a plurality of probes limited to those for identifying at least one transcript of each of the genes in the following first set of markers: AMF, ATF4, Cyr61, ER, Matriptase2, MET, MLN64, MMP7, Nectin4, PAR1A, Psoriason, Pttg1, Rho-C, Scotin, SDF1, SEMP1, SPF45, SST1, ST15, TACC2, TBD10, TCF2, TEM6, TEM7R, and ZO-3;
(b) a plurality of probes for identifying at least one transcript of each of the genes in the following second set of markers ARP2, Atf-3, HuR, MEN1, Paracellin, PTP-RK Radixin, and RHO8/gdiG-Ratio; and
(c) optional reagents and instructions that determine, or show how to determine, the level of expression of each of said genes.

13. A kit according to Claim 12, wherein said kit additionally consists of:
(a) a plurality of probes capable of identifying at least one transcript of the genes in the following expanded first set of markers: Basigin, Beta-catenin, BMP1, BMP10, Calpain large, CD44, CX43, cyclinD2, EHMS, FAK, FAP, GIRK, HAVR1, Isotopo3, JAK1, LOX12, NET-2, PAR1A2, PTHrP, Rho-G, S100A4, SPARC, TCF3, VECAD, Vilip, and Wave2; and
(b) a plurality of probes capable of identifying at least one transcript of the genes in the following expanded second set of markers : aMOT, Atf-1, Claudin-1, IL22R, Rock 2, and VEG1;
(c) optionally, reagents and instructions that determine the level of expression of each of said genes.

14. A kit for determining the likelihood of survival of a patient, which kit consists of:
(a) at least one microarray consisting of at least one set of probes limited to those for identifying the sets of molecular markers that consist of the molecular signatures described in Claims 1-2; and, optionally,
(b) a secondary microarray comprising a plurality of probes for identifying the same set of molecular markers in an internal standard that represents the level of expression of said markers in either a cancer free individual or a patient with a moderate prognosis.

15. A microarray according to claim 14.

16. A set of probes according to claims 12-13.

## Patentansprüche

1. Verfahren zum Bestimmen der Prognose von Säuger-Brustkrebs, wobei das Verfahren umfasst:
(a) Untersuchen einer Probe von Brustkrebsgewebe von einem Lebewesen zum Bestimmen des Expressionsniveaus von Genen, die den folgenden ersten Satz von molekularen Markern: AMF, ATF4, Cyr61, ER, Matriptase2, MET, MLN64, MMP7, Nectin4, PAR1A, Psoriason, Pttg1, Rho-C, Scotin, SDF1, SEMP1, SPF45, SST1, ST15, TACC2, TBD10, TCF2, TEM6, TEM7R und ZO-3, und den folgenden zweiten Satz von molekularen Markern: ARP2, Atf-3, HuR, MEN1, Paracellin, PTP-RK Radixin und RHO8/gdiG-Verhältnis, kodieren und
(b) wenn ein hohes Expressionsniveau für den ersten Satz von molekularen Markern festgestellt wird und ein niedriges Expressionsniveau für den zweiten Satz von molekularen Markern festgestellt wird,
(c) Folgern, dass das Lebewesen, von dem die Gewebeprobe entnommen worden ist, eine geringe Überlebenswahrscheinlichkeit aufweist, die derart ist, dass weniger als 20 % Lebewesen länger als fünf Jahre überleben.

2. Verfahren nach Anspruch 1, bei dem der Teil (a) zusätzlich das Bestimmen des Expressionsniveaus von Genen umfasst, welche den folgenden erweiterten ersten Satz von molekularen Markern: Basigin, Beta-Catenin, BMP1, BMP10, große Untereinheit von Calpain, CD44, CX43, CyclinD2, EHMS, FAK, FAP, GIRK, HAVR1, Isotopo3, JAK1, LOX12, NET-2, PAR1A2, PTHrP, Rho-G, S100A4, SPARC, TCF3, VECAD, Vilip, Wave2, und den folgenden erweiterten zweiten Satz von molekularen Markern: aMOT, Atf-1, Claudin-1, IL22R, Rock 2, Veg1, kodieren.

3. Verfahren nach einem der vorhergehenden Ansprüche, bei dem das Krebsgewebe von einem Menschen ist.

4. Verfahren nach einem der vorhergehenden Ansprüche, bei dem das Krebsgewebe von einem weiblichen Lebewesen ist.

5. Verfahren nach einem der vorhergehenden Ansprüche, bei dem das Expressionsniveau durch Testen bezüglich der Gegenwart von RNA oder mRNA bestimmt wird.

6. Verfahren nach einem der Ansprüche 1 bis 5, bei dem das Expressionsniveau durch Testen bezüglich des Proteins oder der Proteine, das oder die durch die molekularen Marker kodiert wird oder werden, bestimmt wird.

7. Verfahren nach Anspruch 6, wobei das Verfahren die Verwendung von Mitteln umfasst, die an das relevante Protein oder die relevanten Proteine binden und diese(s) so identifizieren.

8. Verfahren nach Anspruch 7, bei dem die Mittel Antikörper sind.

9. Verfahren nach einem der Ansprüche 1 bis 4, bei dem vor dem Durchführen von Teil (a) der ausgewählte Marker amplifiziert wird.

10. Verfahren nach Anspruch 9, bei dem der Marker mittels PCR amplifiziert wird.

11. Verfahren nach einem der vorhergehenden Ansprüche, bei dem das Expressionsniveau eines gegebenen molekularen Markers bezüglich einer Kontrollprobe bestimmt wird, wobei die Kontrollprobe irgendeine der Folgenden ist: eine Probe von Brustgewebe, die frei von Krebs ist, eine Probe von Brustgewebe, die von einem Lebewesen entnommen worden ist, bei dem kein Krebs vorliegt, oder ein anerkannter Standard für die Expression jedes relevanten molekularen Markers in einem gesunden Lebewesen.

12. Kit zum Bestimmen der Prognose von Säuger-Brustkrebs, bestehend aus:
(a) einer Mehrzahl von Sonden, die auf diejenigen zum Identifizieren mindestens eines Transkripts von jedem der Gene in dem folgenden ersten Satz von Markern beschränkt sind: AMF, ATF4, Cyr61, ER, Matriptase2, MET, MLN64, MMP7, Nectin4, PAR1A, Psoriason, Pttg1, Rho-C, Scotin, SDF1, SEMP1, SPF45, SST1, ST15, TACC2, TBD10, TCF2, TEM6, TEM7R und ZO-3,
(b) einer Mehrzahl von Sonden zum Identifizieren mindestens eines Transkripts von jedem der Gene in dem folgenden zweiten Satz von Markern ARP2, Atf-3, HuR, MEN1, Paracellin, PTP-RK Radixin und RHO8/gdiG-Verhältnis, und
(c) optionalen Reagenzien und Anweisungen zum Bestimmen des Expressionsniveaus von jedem dieser Gene oder zum Zeigen, wie dieses zu bestimmen ist.

13. Kit nach Anspruch 12, wobei der Kit zusätzlich besteht aus:
(a) einer Mehrzahl von Sonden, die mindestens ein Transkript von den Genen in dem folgenden erweiterten ersten Satz von Markern identifizieren können: Basigin, Beta-Catenin, BMP1, BMP10, große Untereinheit von Calpain, CD44, CX43, CyclinD2, EHMS, FAK, FAP, GIRK, HAVR1, Isotopo3, JAK1, LOX12, NET-2, PAR1A2, PTHrP, Rho-G, S100A4, SPARC, TCF3, VECAD, Vilip und Wave2, und
(b) einer Mehrzahl von Sonden, die mindestens ein Transkript von den Genen in dem folgenden erweiterten zweiten Satz von Markern identifizieren können: aMOT, Atf-1, Claudin-1, IL22R, Rock 2 und Veg1, und
(c) optional Reagenzien und Anweisungen zum Bestimmen des Expressionsniveaus von jedem dieser Gene.

14. Kit zum Bestimmen der Überlebenswahrscheinlichkeit eines Patienten, wobei der Kit besteht aus:
(a) mindestens einem Mikroarray, der aus mindestens einem Satz von Sonden besteht, die auf diejenigen zum Identifizieren der Sätze von molekularen Markern beschränkt sind, die aus den in den Ansprüchen 1 und 2 beschriebenen molekularen Signaturen bestehen, und gegebenenfalls
(b) einem sekundären Mikroarray, der eine Mehrzahl von Sonden zum Identifizieren des gleichen Satzes von molekularen Markern in einem internen Standard umfasst, der das Expressionsniveau dieser Marker in entweder einem krebsfreien Lebewesen oder einem Patienten mit einer mäßigen Prognose darstellt.

15. Mikroarray gemäß Anspruch 14.

16. Satz von Sonden nach den Ansprüchen 12 und 13.

## Revendications

1. Procédé pour déterminer le pronostic d'un cancer du sein chez un mammifère, lequel procédé comprend :
(a) l'examen d'un échantillon de tissu cancéreux du sein provenant d'un individu de façon à déterminer le taux d'expression de gènes codant pour le premier ensemble de marqueurs moléculaires suivants : AMF, ATF4, Cyr61, ER, Matriptase2, MET, MLN64, MMP7, Nectine-4, PAR1A, Psoriason, Pttg1, Rho-C, Scotine, SDF1, SEMP1, SPF45, SST1, ST15, TACC2, TBD10, TCF2, TEM6, TEM7R et ZO-3 ; et le second ensemble de marqueurs moléculaires suivants : ARP2, Atf-3, HuR, MEN1, Paracellin, PTP-RK Radixin, et RHO8/gdiG-Ratio et
(b) si un taux d'expression élevé est déterminé pour le premier ensemble de marqueurs moléculaires et un taux d'expression bas est déterminé pour le second ensemble de marqueurs ;
(c) conclure que l'individu à partir duquel l'échantillon de tissu a été prélevé a une faible probabilité de survie qui est inférieure à 20 % de la survie d'individus de plus de cinq ans.

2. Procédé selon la revendication 1, dans lequel la partie (a) comprend en outre la détermination du taux d'expression de gènes codant pour le premier ensemble élargi de marqueurs moléculaires suivants : Basigine, Béta-caténine, BMP1, BMP10, Calpaïne large, CD44, CX43, cycline-D2, EHMS, FAK, FAP, GIRK, HAVR1, Isotopo3, JAK1, LOX12, NET-2, PAR1A2, PTHrP, Rho-G, S100A4, SPARC, TCF3, VECAD, Vilip, Wave2, et le second ensemble élargi de marqueurs moléculaires suivants : aMOT, Atf-1, Claudin-1, IL22R, Rock2, Veg1.

3. Procédé selon l'une quelconque des revendications précédentes, dans lequel le tissu cancéreux provient d'un être humain.

4. Procédé selon l'une quelconque des revendications précédentes, dans lequel le tissu cancéreux provient d'une femme.

5. Procédé selon l'une quelconque des revendications précédentes, dans lequel le taux d'expression est déterminé par essai pour la présence d'ARN ou d'ARNm.

6. Procédé selon l'une quelconque des revendications 1 à 5, dans lequel le taux d'expression est déterminé par essai pour la ou les protéines codées par les marqueurs moléculaires.

7. Procédé selon la revendication 6, dans lequel le procédé met en jeu l'utilisation d'agents qui se lient à la ou aux protéines pertinentes et identifient ainsi celles-ci.

8. Procédé selon la revendication 7, dans lequel les agents sont des anticorps.

9. Procédé selon l'une quelconque des revendications 1-4, dans lequel avant d'effectuer la partie (a), le marqueur sélectionné est amplifié.

10. Procédé selon la revendication 9, dans lequel le marqueur est amplifié par PCR.

11. Procédé selon l'une quelconque des revendications précédentes, dans lequel le taux d'expression d'un marqueur moléculaire donné est déterminé ayant trait à un échantillon témoin, l'échantillon témoin étant l'un quelconque des suivants : un échantillon de tissu de sein qui est exempt de cancer, un échantillon de tissu de sein prélevé sur un individu qui ne présente pas de cancer, ou un étalon reconnu pour l'expression de chaque marqueur moléculaire pertinent dans un individu sain.

12. Coffret pour déterminer le pronostic d'un cancer du sein chez un mammifère, qui consiste en :
(a) une pluralité de sondes limitées à celles pour identifier au moins un produit de transcription de chacun des gènes dans lé premier ensemble de marqueurs suivants : AMF, ATF4, Cyr61, ER, Matriptase2, MET, MLN64, MMP7, Nectine-4, PAR1A, Psoriason, Pttg1, Rho-C, Scotine, SDF1, SEMP1, SPF45, SST1, ST15, TACC2, TBD10, TCF2, TEM6, TEM7R et ZO-3 ; et
(b) une pluralité de sondes pour identifier au moins un produit de transcription de chacun des gènes dans le second ensemble de marqueurs suivants : ARP2, Atf-3, HuR, MEN1, Paracellin, PTP-RK Radixin, et RHO8/gdiG-Ratio, et
(c) facultativement, des réactifs et des instructions qui déterminent, ou montrent comment déterminer, le taux d'expression de chacun desdits gènes.

13. Coffret selon la revendication 12, dans lequel ledit coffret consiste en outre en :
(a) une pluralité de sondes capables d'identifier au moins un produit de transcription des gènes dans le premier ensemble élargi de marqueurs suivants : Basigine, Béta-caténine, BMP1, BMP10, Calpaïne large, CD44, CX43, cycline-D2, EHMS, FAK, FAP, GIRK, HAVR1, Isotopo3, JAK1, LOX12, NET-2, PAR1A2, PTHrP, Rho-G, S100A4, SPARC, TCF3, VECAD, Vilip, Wave2 et
(b) une pluralité de sondes capables d'identifier au moins un produit de transcription des gènes dans le second ensemble élargi de marqueurs suivants : aMOT, Atf-1, Claudine-1, IL22R, Rock2 et VEG1;
(c) facultativement, des réactifs et des instructions qui déterminent le taux d'expression de chacun desdits gènes.

14. Coffret pour déterminer la probabilité de survie chez un patient, lequel coffret consiste en :
(a) au moins un microréseau consistant en au moins un ensemble de sondes limitées à celles pour identifier les ensembles de marqueurs moléculaires qui consistent en les signatures moléculaires décrites dans les revendications 1-2 ; et, facultativement,
(b) un microréseau secondaire comprenant une pluralité de sondes pour identifier le même ensemble de marqueurs moléculaires dans un étalon interne qui représente le taux d'expression desdits marqueurs soit dans un individu exempt de cancer, soit dans un patient présentant un pronostic modéré.

15. Microréseau selon la revendication 14.

16. Ensemble de sondes selon l'une des revendications 12 et 13.
